# EUROPEAN PATENT APPLICATION

(11) **EP 4 068 300 A1**
(43) Date of publication of application: **05.10.2022**
(21) Application number: 21166520.3
(22) Date of filing: 01.04.2021
(51) Int. Cl.: G16H 30/20, G16H 40/63, A61B 6/03, A61B 6/00

(54) **METHOD, MEDICAL IMAGING DEVICE AND CONTROL UNIT FOR PERFORMING A MEDICAL WORKFLOW**

(71) Applicant: Siemens Healthcare GmbH, 91052 Erlangen (DE)
(72) Inventor: Gutjahr, Ralf, 90459 Nürnberg (DE); Sahbaee Bagherzadeh, Pooyan, Mount Pleasant, SC 29466 (US); Schmidt, Bernhard, 90766 Fürth (DE)

(57) **Abstract**

The invention concerns a method for performing a medical workflow comprising a diagnostic scan of a body part of a patient (9) with a medical imaging system (2), in particular a computed tomography system, comprising a scanner, a control unit (1) and at least one camera, in particular a 3D camera, the method comprising the steps: (a) automatically monitoring the status of at least some of the system's components and transferring said status information to the control unit (1); (b) acquiring images of the patient (9) with the at least one camera and interpreting the images by the control unit (1) in order to detect at least one aspect of the patient's condition; (c) determining scan parameters for the diagnostic scan based on the at least one aspect of the patient's condition and/or the status of the system's components; (d) automatically determining a suitable amount of a contrast medium required for the diagnostic scan and/or an administration time of the contrast medium with respect to the scan, based on the at least one aspect of the patient's condition, by the control unit (1); (e) transferring the determined scan parameters from the control unit (1) to the scanner for performing the diagnostic scan, and optionally controlling an injector to inject contrast medium at the determined amount and/or administration time. The invention further concerns a corresponding medical imaging system and a control unit.

## Description

The invention relates to a method for performing a medical workflow, a medical imaging system, and a control unit for a medical imaging system.

Diagnostic imaging is an important factor for finding a diagnosis and treatment for many diseases. Imaging exams, such as computed tomography (CT) examinations, but also examinations with other imaging modalities such as Magnetic Resonance Imaging (MRI) or Positron Emission Tomography (PET), typically comprise many different work steps which are carried out by one or several persons. For example, technicians ("techs"), in particular radiology technicians, are performing the patient positioning, contrast administrations, and/or set-up of monitoring devices. Furthermore, they have to ensure that all the equipment, lines, and cables are not interfering with the imaging procedure. Additionally, prior to the acquisition of medical images, various information about the patient's condition need to be evaluated, in particular by physicians, techs, nursing staff or other clinical staff members. For example, information is obtained during anamnesis (e.g., medical history, genetics, allergies, etc.) or via direct measures (e.g., temperature, ECG, laboratory findings, etc.). These sources of information are for example used to set up a suitable scan protocol and/or contrast administrations protocol.

Consequently, these sources of information are often collected by different entities (e.g., tech, radiologist, scanner, injector) and at different moments in time (e.g., at different points in time prior to the examination as well as during examination). Therefore, the quality of the imaging, and hence diagnosis, currently strongly relies on the expertise of the team of technicians and other clinical staff as well as on their coordination. In emergency situations (e.g., trauma) fast and proper communication between these entities cannot always be guaranteed, which can lead to either slow examination set ups or sometimes selection of wrong parameters and therefore failures and in the worst case to nondiagnostic images and harm to the patient.

In the state of the art, the problem of a slow examination setup is sometimes approached by applying a nonindividualized setup where the patient's characteristics are not considered. However, this may result in unnecessarily high radiation doses and/or in suboptimal administration of contrast media, which can both bring harm to the patient (e.g., extravasation, such as leakage of blood from a vessel, and/or acute kidney injury) and might lead to nondiagnostic images (e.g., due to wrong scan timing and/or image artefacts). On the other hand, measures are applied to adapt the contrast injection protocols, e.g. by manually scaling the amount of applied contrast medium volume to the patient's weight or by selecting a suitable contrast medium concentration depending on the clinical question. However, these measures are typically time consuming and error prone due to user interaction, since expertise and time are required to properly understand, configure and apply the various techniques. In particular in urgent clinical scenarios, technical experts might not be available and time can be critical.

It is therefore an object of the invention to provide means to improve a workflow concerning medical imaging with regard to time consumption and/or reliability.

This object is achieved by a method for performing a medical workflow according to claim 1, a medical imaging system according to claim 13 and a control unit according to claim 15. Further advantages and features of the invention result from the sub-claims as well as the description and the attached figures.

According to a first aspect of the invention, a method for performing a medical workflow is provided, the workflow comprising a diagnostic scan of a body part of a patient with a medical imaging system, in particular a computed tomography system, comprising a scanner, a control unit and at least one camera, in particular a 3D camera, the method comprising the steps: (a) automatically monitoring the status of at least some of the system's components and transferring said status information to the control unit; (b) acquiring images of the patient with the at least one camera and interpreting the images by the control unit in order to detect at least one aspect of the patient's condition; (c) determining scan parameters for the diagnostic scan based on the at least one aspect of the patient's condition and/or the status of the system's components; (d) automatically determining a suitable amount of a contrast medium required for the diagnostic scan and/or an administration time of the contrast medium with respect to the scan, based on the at least one aspect of the patient's condition, by the control unit; (e) transferring the determined scan parameters from the control unit to the scanner for performing the diagnostic scan.

In this context a medical workflow is a series of steps, in particular the steps mentioned above and optionally further steps, which are carried out in sequence or (partially) simultaneously in order to provide or help to provide a medical diagnosis and/or contribute to the treatment of the patient. The steps may in particular be carried out in the given order. However, it is also conceivable that the order may be changed with respect to some steps and/or that some steps are carried out simultaneously. For example, steps (c) and (d) may be exchanged with respect to their sequence or carried out simultaneously. The same may apply to steps (a) and (b). In preferred embodiments, at least some or all of the steps are carried out automatically, i.e. without requiring user interaction. In other embodiments, the user (e.g. the tech) has to trigger some steps, but does not have to provide substantive input.

The body part of the patient may for example be an organ, a vessel, e.g. a blood vessel or a lymphatic vessel, a limb or part of a limb. It may also be the head. The diagnostic scan may in particular be done with a medical imaging modality, such as x-ray imaging, computed tomography (CT) imaging, magnetic resonance imaging or ultrasound imaging. The control unit may for example be a computer or part of a computer or control station. The at least one camera is preferably optical camera, it may be a 3D camera. The at least one camera may for example be located at the ceiling, on a frame structure near the ceiling and/or be attached to another component of the medical imaging system. Advantageously, this method may provide an automated and well-coordinated medical workflow, wherein a patient's needs and/or characteristics may be individually regarded while simultaneously enabling time efficiency and improved consistency as well as taking interand intra-institutional standards into account. In particular an automated coordination with the control unit may allow for the method being less dependent on individual expertise and experience of clinical personnel and less prone to human errors. In particular the interplay of the system's single components may advantageously be considered, in particular by the control unit, which may lead to an improved workflow.

The status of the system's components may in particular be monitored via the at least one camera and/or via other monitoring devices such as a heat sensor and/or internal checkup functions of the individual components. In particular, such monitoring devices may check the availability of certain imaging functions. Alternatively or additionally, the camera is used in order to acquire images (e.g. photos or video footage) of the patient. The images may in particular be sent to the control unit for interpretation. Aspects of the patient's condition may for example be vital functions, e.g. a breathing rhythm, respiratory information, a breathing amplitude, the heart rate or pulse, a body temperature and/or cardiac output. Alternatively or additionally, aspects of the patient's condition may be attempts for nonverbal communication, in particular if the patient cannot articulate himself/herself, such as gestures, facial expressions, in particular indicating pain, and/or movement of the patient's mouth. Further possible options with regard to the detection of the patient's condition are the detection of an extravasation, e.g. an open wound or hematoma, in particular by using a 3D camera, the detection of blood stains on the system, in particular with regard to hygiene and the safety of the operational staff, a detection of an extremity positioning and posture, in particular including arm positioning, and/or the detection of patient movement between scans. The positioning and posture may for example relevant for the diagnostic scan, wherein extremities may obstruct the scanner's view. The control unit may be adapted to send a notification when a change of posture is necessary, e.g. when arms should be held up or down, in particular depending on the applied scan protocol. The detection of patient movement may for example trigger a registration by the control unit, wherein the control unit may initiate an adaption of scan parameters, such as an adaption of the scan area, in particular of a region of interest to be scanned.

In order to determine the scan parameters and/or for the determination of the amount and administration time of the contrast medium, the control unit may further take into account scanner specific properties, optionally with regard to the properties of other components, such as a contrast medium injector, and/or with regard to a venous access and/or properties of the body part, in particular a target organ. Additionally or alternatively, the control unit may determine the scan parameters based on the amount and/or administration time of the contrast medium and/or additional contrast medium related parameters, e.g. a flow rate, a volume, a saline flush and or a viscosity. Scan parameters may in particular comprise one or several of a scan timing and or scan rate, a scan duration, an MR imaging protocol/sequence, a scan direction, tube voltages, tube currents, energy threshold selection for photon counting CT scanners, determination of the scan range, an ECG-gating, a selected collimation, minimizing radiation dose, e.g. via a dose modulation, and a dual- or multi-energy parametrization. An automatic selection of collimation may include a flying focal spot, i.e. the variation of the scanner's beam path through the body part of the patient, and/or a comb filtration to increase the detector's spatial resolution. In particular for radiation-based, such as x-ray based, imaging systems, tube voltages and tube currents may be adapted and/or a dose modulation may be used to optimize the radiation dose. The radiation dose may be controlled by controlling the tube current, i.e. the stream of electrons between the cathode and the anode of an x-ray generating unit. The tube current may be modulated with respect to the tube angle and/or with respect to a longitudinal direction, in particular in order to adapt the radiation dose for different parts of the patient's body. An automatic selection of a dual- or multi-energy parametrization may be applied when using spectral computed tomography (CT) systems with multi-energy CT technologies. It may in particular be based on the patient's size, age and/or profile. Applicable multi-energy techniques may be a dual spiral technology or a dual source dual energy technology or a slow kV switching technology or a fast kV switching technology or a dual layer CT or a photon counting CT, wherein tube voltages and/or tube currents, in particular including automatic exposure control, are selected automatically. Furthermore, the number of energy thresholds and/or energy threshold values may be selected.

According to an embodiment, the control unit may in particular control the administration of the contrast medium via an injector and/or transfer an injection protocol, including the amount and administration time of the contrast medium, to the injector. Accordingly, the method may include a step of the control unit controlling a contrast injector to inject contrast medium into the patient at the determined amount and/or administration time. In addition to determining the amount and administration time of the contrast medium the control unit may optionally determine an injection flow rate and an injection duration. In case of oral administration of contrast medium, the control unit may display the determined and/or required amount to the user.

The method may further comprise one or several of the additional steps: administering the contrast medium, in particular via an automatic injector, based on the determined amount and administration time; the scanner performing the diagnostic scan based on the determined scan parameters; automatically selecting image reconstruction parameters, in particular orientations, reconstruction methods, filtration, iterative reconstruction strength level, slice increment, and/or slice width.

According to an embodiment, it may be provided that the at least one camera acquires images (e.g. photos or videos) of the patient's environment throughout the scanning session. The images may in particular be sent to the control unit and the control unit may use the images for a scene interpretation. Advantageously, the camera may be a 3D camera. A 3D camera may in particular allow to interpret the scene with greater detail due to the additional depth-related information. Furthermore, the monitoring may comprise checking for deviations from a predetermined scanning workflow and, in the case of detected deviations, sending an alarm to a user and/or initiating counter measures and/or stopping the workflow. In particular the images may be sent to the control unit and the checking for deviations may be carried out by the control unit based on the images. Alternatively or additionally the control unit may check for deviations based on additional control elements such as sensors and/or internal system check-up functions, in particular of the scanner and/or an contrast medium injector. Deviations from the workflow may for example be malfunctioning or non-functioning components or unusual occurrences, such as blood or iodine dripping, something accidentally falling down or the patient tripping and/or falling. Using the camera, in particular the 3D camera, may advantageously allow the system to be ready at any time and able to adapt to various scenarios. This may in particular be advantageous in emergency situations, wherein significantly less user input may be needed because the system is able to recognize the situation automatically due to the information obtained from the camera images.

According to an embodiment, the medical imaging system may comprise a contrast injector adapted to inject the contrast medium into a patient at an injection site, and the monitoring may comprise measuring the temperature of the contrast medium load in the injector and sending the measured value to the control unit. In particular monitoring the status of at least some of the system's components may comprise automatically measuring the temperature of the contrast medium with a temperature controller and sending the measured temperature to the control unit. The control unit may compare the measured temperature with a predetermined threshold value stored on the control unit and output an alarm if the temperature is below the predetermined threshold value. A preheated contrast medium may have improved pharmacokinetic properties when compared to a cold contrast medium. Hence the control unit may send a warning to the user if the contrast medium is not warm enough for optimal application. The threshold value may for example be in the range of 32°C to 38 °C, preferably 34°C to 36°C. Monitoring the temperature may thus avoid the erroneous and disadvantageous application of cold contrast medium.

According to an embodiment, the monitoring may comprise checking for an extravasation and, in the case of detecting an extravasation, stopping the workflow and/or outputting an alarm. The extravasation may for example be a vessel rupture, an open wound or a hematoma. An extravasation may lead to a swelling of the corresponding area. The detection may for example be possible via the camera, in particular 3D camera, wherein images sent to the control unit are checked by the control unit for indications of an extravasation, e.g. for corresponding image patterns concerning forms and/or colours. Advantageously, the possibility to automatically detect an extravasation may enable the medical staff to react to a critical condition of the patient in time , which otherwise might be detected too late, in particular not before the end of the imaging process, and/or to note an occurrence that might interfere with the scan quality.

According to an embodiment, the patient may be identified by visually detecting the patient via the at least one camera and by comparing the visuals of the patient to a data base. Visually detecting may in particular comprise taking image data, e.g. a picture or video recording, of the patient, in particular of the patient's face. For example, the at least one camera may comprise a face recognition or face detection algorithm in order to allow an automatic focusing on the patient's face. The image data may consecutively be transferred to the control unit. In this context the term "visuals of the patient" may be understood to be a picture or video of the patient's appearance, in particular the patient's face. The control unit may then connect to a data base, e.g. online or within an internal network of the medical facility, in order to run a face recognition algorithm, wherein the image data of the patient are compared to images in the data base. The data base may in particular comprise a modality work list (MLW). For example, the data base may allow to draw additional information about the patient. The data base may for example be part of a radiological information system (RIS). Additionally or alternatively, the control unit or the medical imaging system may also comprise an internal patient data base which is contacted may be used by the control unit. The internal patient data base may for example allow a faster access to the respective patient file.

According to an embodiment, the method may comprise retrieving patient information comprising health parameters from a patient data base by the control unit, wherein the patient information is automatically provided to a user, wherein optionally suggestions for precautious actions are provided to the user. The patient data base may in particular be the hospital information system (HIS) or radiological information system (RIS), wherein the RIS may comprise a modality work list (MWL) containing information about the patient's scheduling. Prior to retrieving information, the patient may either be recognized automatically via the at least one camera and/or the patient may be identified via user input. The user may be a clinical staff member, in particular a medical technologist, a radiologist or a physician. The information may e.g. be provided via a screen and/or via an audio output device and/or via a printout device. The provided information may comprise one or several of the following list: IV (intravenous) contrast allergy, oral contrast preparation, medication allergy, kidney disease failure, dialysis, Thyroid cancer or hyperthyroidism, decreased cardiac output, altered blood pressure, infections, lab values for kidney function, in particular a blood urea nitrogen (BUN) value, a serum creatinine value, and/or an (e)GFR, lab values assessing blood coagulation, in particular a Prothrombin time (PT), a partial thromboplastin time (PTT), and/or a Platelet count, and the patients size, age and/or profile. The automatic download of information may help to save time during an examination because the medical staff does not need to manually retrieve the required information. The suggestions may in particular depend on the health information. The suggestions may comprise: suggesting anticoagulation medication, i.e. blood thinners, e.g. coumadin, heparin, Plavix and/or aspirin, and/or suggesting hydration for patients with renal insufficiencies, e.g. increased serum creatinine or decreased (e)GFR). Providing these suggestions may help to minimize the occurrence of human errors during the workflow. Furthermore, the control unit may provide an online help connection to experienced technicians or to a centralized scan support, e.g. in the form of a remote control support such as the "virtual cockpit" by Siemens. Additionally or alternatively the information provided, e.g. on a screen, may comprise the remaining scan time, in particular the time till the entire scan is finished, and or an automatically generated scan and patient specific procedure checklist and/or progress overview.

According to an embodiment, determining the amount and/or administration time and/or flow rate of the contrast medium may be based on one or more of the patient's height, weight, body surface area, body volume, body mass index, size of the body part, in particular an organ size, the position of the injection site, the used cannula, the temperature of the contrast medium, the patient age, the set scan duration and/or the patient's clinical indication. The determination of the amount of the contrast medium may in particular be carried out automatically by the control unit. The body volume and/or surface area may for example allow a more accurate and/or better estimate of an appropriate amount or volume of the contrast medium volume than solely the patient's body weight. On the other hand, the height and weight may have the advantage to be easier to determine. According to a more specific embodiment, the method may comprise retrieving patient information from a patient data base by the control unit, wherein the patient information comprises at least one of the patient's height, weight, body surface area, body volume, body mass index and size of the body part, in particular an organ size, and wherein said patient information is used in determining the amount and/or the administration time and/or the flow rate of the contrast medium. Additionally or alternatively a detection mechanism at the injector may automatically monitor that a saline chaser is used and optionally send out an alarm if a saline chaser is not used or if there is no sufficient amount of saline in or at the injector.

According to an embodiment, the method may comprise automatically performing a scout scan prior to the diagnostic scan, wherein the field-of-view and the scan parameters of the scout scan are determined based on the at least one aspect of the patient's condition and/or the status of the system's components and/or on patient information retrieved from a data base. The scout scan may for example be used to check the size and or the position of an organ, such as the lung. The scout scan may in particular be understood to be a topogram, which is an overview projection image acquired with a CT scanner in order to be able to plan and position the CT slices to be acquired in the subsequent scans, e.g. pre-monitoring scan, a monitoring scan and/or a diagnostic scan. The patient's condition may for example comprise the patient's height, weight, body surface area, body volume, body mass index and size of the body part, in particular an organ size, wherein the position of the field-of-view and the necessary intensity of the scan may be based on the geometry of the patient's body and the body part to be scanned. The scout scan may be used to determine the position of a consecutive scan, e.g. a diagnostic scan and/or a further monitoring scan, such as a single-slice image.

According to an embodiment, the method may comprise the additional steps: acquiring a topogram of the body part via the scanner using a low radiation dose, and automatically determining a pre-monitoring slice position based on the topogram and/or on image data from the at least one camera; and pre-monitoring the body part by acquiring a single-slice image of the body part. Pre-monitoring includes the acquisition of low radiation dose images in an area which will be flooded with the contrast medium just before the region of interest. A time sequence of such images will be acquired until the contrast medium concentration begins to rise, for example until a threshold value in HU is reached. Then, the diagnostic images are with higher radiation dose are acquired, for example a time sequence of images with which the contrast medium invasion (flooding) and outflow can be monitored. This technique is also called bolus tracking. Accordingly, if blood flow in the heart or the coronary arteries is to be studied, the pre-monitoring slice may be positioned at the tip of the heart. The radiation dose may be based on the patient's height, weight, body surface area, body volume, body mass index and size of the body part, in particular an organ size, wherein these parameters may be determined via the at least one camera and or via retrieved patient information and or by user input. The pre-monitoring may in particular carried out at a low radiation dose, such that no or barely any diagnostic value may be gained but a correct start time of the monitoring images may be identified. According to an embodiment, the method may be provided, wherein a minimal radiation dose for the acquisition of the topogram is determined by evaluating images of the patient obtained by the at least one camera. According to an embodiment, the pre-monitoring slice position may be further based on images of the patient taken by the at least one camera and/or landmark detection techniques and/or the patient's height, weight, body surface area, body volume and/or body mass index. Landmarks may for example be anatomical body parts, observed in the topogram, wherein the landmarks may be determined with landmark detection techniques, e.g. with the Siemens auto ROI tool automatically determining the location and/or size of the region-of-interest. The landmark detection technique may be based on a recognition of anatomical body parts, such as ascending aorta, descending aorta, pulmonary artery, carotids and/or femoral arteries. The system may automatically store the image data from the camera and the topogram data, in particular register the camera image data to the topogram. Storing and registration of the data may help to reduce the additional radiation dose for future examinations, i.e. rescans of the patient.

According to an embodiment, the scan rate or a sampling frequency of the diagnostic scan may be adjusted according to different phases of contrast medium invasion into the part of the patient's body. Adjusting the scan rate or sampling frequency may for example mean, that a lower scan rate is applied as long as the flow of the contrast medium has not yet reached the body part to be examined. E.g. an automatic vessel tracking may be applied, wherein the system automatically recognizes the current location of the contrast medium. The scan rate may be increased as soon as the contrast medium reaches the body part. Therefore, the number of scans, in particular monitoring scans, and thus the overall radiation dose may advantageously be reduced. This embodiment can for example be applied in the context of Bolus Tracking. Additionally or alternatively the scan intensity, e.g. the radiation dose may be adjusted accordingly as well. This may lead to an even lower radiation dose, wherein the image quality is lower in the monitoring phase before the contrast medium reaches the body part.

According to an embodiment, the scanning may comprise acquiring a time series or time sequence of images at a region-of-interest, and wherein the position of the region-of-interest may be automatically adjusted between images to correct for patient movement, as determined by the at least one camera. Tracking the patient's movement with the at least one camera may allow for fewer scans, in particular scouting scans, without losing track of the positioning of the patient. Hence, this may allow to further reduce the radiation dose.

According to an embodiment, the method may comprise automatically storing documentation data of the workflow, the documentation data including the camera images or their interpretation and/or quality parameters of a contrast medium injection, including injection pressure and optionally contrast medium temperature. For example, the camera images, e.g. 2D or 3D camera images, may be registered to an acquired topogram. Storing the documentation data may on the one hand allow using these data for future examinations of the same patient, thus potentially reducing the future radiation doses, and/or these data may be used for documentation, in particular allowing to reconstruct the workflow of the examination at a later time and/or apply some additional analysis. Furthermore, using the same topogram may help to achieve a better consistency between different scans. For example, the quality of the single steps, e.g. an injection quality and/or a documentation whether warm or cold contrast medium was used, of the workflow may be assessed retrospectively. Additionally or alternatively, the anonymized 3D camera recordings or a semantic interpretation may be provided. According to an embodiment, the method may comprise automatically selecting image reconstruction parameters and/or checking the system status. Image reconstruction parameters may for example be orientations, applied reconstruction methods, filtration, iterative reconstruction strength level, slice increment, and/or slice width. An automatic selection of image reconstruction parameters may allow for a faster access to images to be analyzed for a diagnosis. The system status may in particular be checked with respect to the ability to conduct further examinations and/or scans. For example, it may be checked, whether consumable materials, e.g. the loaded contrast medium, are still there in sufficient amounts. Optionally a warning may be sent to a user, if the system status turns out not to be ready and/or missing consumable materials.

According to another aspect, a medical imaging system, in particular a computed tomography system, is provided, which is adapted to carry out a medical workflow comprising a diagnostic scan of a body part of a patient, in particular according to the method described above, the system comprising: a control unit in communication with at least some of the other components of the system, wherein the control unit is configured to monitor and control the medical workflow, and configured to provide information concerning the medical workflow and the patient to a user and/or to the patient, wherein the control unit is configured to receive and forward and/or apply user input, in particular input commands and/or input information; a scanner adapted to acquire images of a patient's body or of parts of a patient's body; at least one camera, in particular a 3D camera, wherein the at least one camera is configured to provide images of the system environment and/or of the patient to the control unit; wherein the control unit is configured to provide the scanner with scan relevant information and/or to control the scanner. All the features and advantages of the method may be analogously applied to the system and vice versa. The control unit may for example be configured to provide information about the procedure, e.g. the remaining duration of the examination, to the patient. The control unit may be configured to provide patient information, e.g. age, weight, height, and/or lab values, and/or provide warnings, e.g. if patient posture does not correspond to a scan protocol and/or if required patient information, such as kidney function values, is not available, and/or provide information about vital functions and/or a status of the patient and/or about a hygiene condition, e.g. blood stains, and/or irregularities in the workflow and/or with the system's components, and/or used material, and/or provide support with workflow specific information, in particular for unexperienced users, to the technological staff. The scanner may in particular be configured to receive information from the control unit, e.g. a monitoring slice position and/or information about patient movement, in particular how to adjust the scan area, and/or MWL information of the patient. On the other hand, the scanner may be configured to provide information about the estimated scan duration to the control unit. Images of the system environment and/or the patient may comprise one or several of: information for scene interpretation, information to determine optimal monitoring slice position, information to detect an injection site, information to detect a patient posture, information to detect extravasation observation of movement, in particular of the patient during and between the scans. Optionally, the information provided by the camera may be three-dimensional information, wherein the camera is a 3D camera. The control unit may be configured to steadily check the camera function and/or connection. Advantageously, the control unit may be adapted to work as a joint centralized unit, which is capable of coordinating and interpreting various components of the system and/or various sources of information. The control unit may allow a high level of automation, which may support a workflow that is individualized to the patient's needs and properties, while at the same time allowing a time efficient workflow. By being configured to provide information to the users, e.g. the radiologists, the control unit may enable the users to still overview and, if necessary, control the various involved technical entities. In particular the control unit may be configured to control and manage different components, wherein the different components may be provided with different connections and/or interfaces. In other words, the control unit may comprise different standards of interfaces and/or connections, e.g. ethernet connections and serial interfaces. The control unit may advantageously allow to connect multiple devices to the scanner, even though the scanner itself does not have enough connections to be connected to all devices at once.

According to an embodiment, the system may further comprise a contrast injector adapted to inject a contrast medium into a patient at an injection site, wherein the contrast injector is configured to measure the temperature of the contrast medium and send the measured value to the control unit, wherein the control unit is configured to control functions of the contrast injector and/or provide information concerning the contrast medium injection to the contrast injector. For example the control unit may be configured to provide information concerning one or several of: an injection site, MWL information about the patient, information about the patient's height, weight, body mass index, and/or body surface area, in particular for contrast medium volume scaling. The control unit may additionally and/or alternatively be configured to stop an injection signal, in particular in case of an emergency occurrence, such as a detected extravasation, and or send a control signal for warming the contrast medium. The injector may further be configured to provide injection information to the control unit, wherein the control unit may be configured to evaluate the injection information to determine faults, e.g. an exceeded pressure limit and/or irregularities, which may be caused by an extravasation and/or air bubbles. According to an embodiment, the contrast injector may be configured to measure the temperature of the contrast medium and send the measured temperature value to the control unit, wherein the control unit is configured to compare the measured temperature value to a predetermined minimal value and, in case the predetermined minimal value is above the measured temperature value, to stop the workflow and/or output an alarm.

According to another embodiment, the system may comprise and/or be connected to a patient data base, wherein the patient data base comprises information about health parameters of a plurality of patients, wherein the control unit is configured to retrieve patient information comprising health parameters from the patient data base. The patient data base may in particular be or be part of a hospital information system (HIS) or radiological information system (RIS).

According to another aspect, a control unit for a medical imaging system, in particular a medical imaging system according to one of the claims, is provided, wherein the control unit is configured to monitor and control a medical workflow, wherein the control unit is configured to provide information concerning the medical workflow and a patient to a user and optionally to the patient, wherein the control unit is configured to provide a scanner of the medical imaging system with scan-relevant information, in particular scan parameters, wherein the control unit is optionally configured to control functions of a contrast injector that is part of the medical imaging system and/or provide information concerning a contrast medium injection to the contrast injector. All the features and advantages of the method and/or the system may be analogously applied to the control unit and vice versa. According to an embodiment, the control unit may be configured to receive images from a camera throughout the workflow, and interpret the images to monitor and control the workflow.

Embodiments of the invention are now described with reference to the attached figures. Similar or corresponding components are designated with the same reference signs.
- Fig. 1: shows a medical imaging system according to an embodiment of the invention,
- Fig. 2: shows a schematic overview of the working principle of the control unit within the medical imaging system according to an embodiment of the invention, and
- Fig. 3: shows a diagram of a method according to an embodiment of the invention.

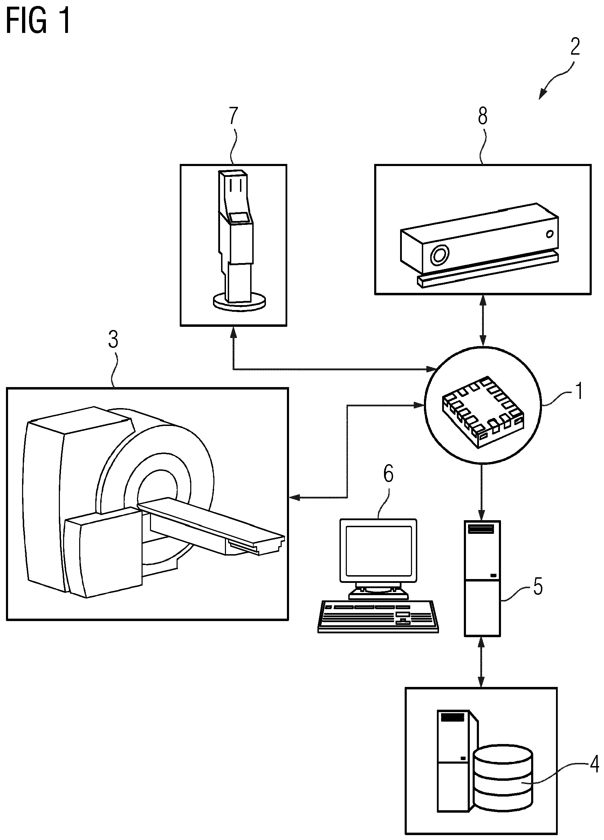

Figure 1 shows a medical imaging system 2 according to an embodiment of the invention. The medical imaging system 2 comprises a control unit 1 which is in communication with other components of the system. The control unit is part of a computer 5 and configured to monitor and control the workflow and may provide information concerning the workflow and/or the patient to a user, in particular a clinical staff member via a screen 6. Additionally, the control unit may provide information to a patient, who is lying on a patient table of a medical imaging device 3, on a separate screen or via audio output. The control unit 1 is further configured to provide scan parameters to a scanner of the medical imaging device 3, in this case a computed tomography device. A 3D camera 8 is configured to provide images of the system environment and the patient to the control unit 1. The medical imaging system 2 further comprises a contrast injector 7 which is also in communication with the control unit 1. For example, the contrast injector may provide a temperature of the contrast medium to the control unit 1, while the control unit 1 may provide parameters concerning the administration of the contrast medium, such as an amount and/or an administration time of the contrast medium, to the injector 7. The control unit 1 is further connected to a data base 3 which contains patient information. The control unit 1 is configured to retrieve patient information, in particular health related information and information about the patient's identity, form the data base 4.

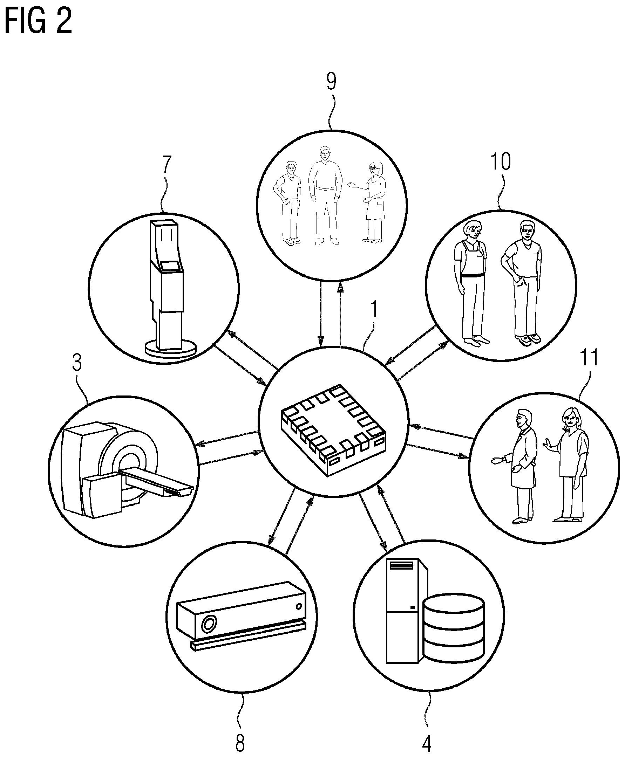

Figure 2 shows a schematic overview of the working principle of the control unit 1 within the medical imaging system according to an embodiment of the invention. The overview shows in particular the connection of the control unit 1 with involved persons and system components. With respect to a patient 9, the control unit 1 is configured to acquire prior information of individual patient characteristics, such as age, weight, height, lab values, etc. On the other hand, the control unit may provide information about the procedure, in particular the scanning or examination procedure, a remaining time, etc. to the patient 9. Providing information to the patient 9 may for example help to comfort the patient 9 during the process. The tech staff 10 may access and, if necessary, adjust the control unit 1. For example, the tech staff 10 may train the control unit occasionally or constantly. On the other hand, the control unit may provide patient information to the tech staff 10, which may in particular be always visible on a screen 6. The control unit 1 may further send a warning, if a patient posture does not correspond to a loaded scan protocol or if required patient information, e.g. kidney function values, is not available. The control unit 1 may further support the tech staff 10 with workflow specific information, such as live guidance for unexperienced users. Additionally the control unit 1 may inform the tech staff 10 about vital functions and/or a status of the patient and/or about a hygiene condition, in particular within the medical imaging device 3, such as blood stains. A currently responsible radiologist 11 may program the control unit 1, while on the other hand the control unit 1 may inform the responsible radiologists 11 about irregularities of the medical imaging system 2 and/or the patient 9, in particular during the workflow. Furthermore, the control unit may inform the radiologists 11 about used material, e.g. for billing and/or restoring purposes. With respect to a connected data base 4, the control unit may retrieve MWL (modality work list) information for the scanner, in particular a CT scanner, of the medical imaging device 3 and for the contrast injector 7. Furthermore, the control unit 1 may carry out a lookup of the recognized patient in a radiology information system of the data base 4. The 3D camera 8 may provide 3D information to the control unit 1, in particular for scene interpretation. for patient recognition and/or identification, to determine an optimal monitoring of a slice position, to detect an injection site, i.e. a position of the injection at the patient, to detect a patient posture, and/or to detect an extravasation at the patient 9. Furthermore, the control unit 1 may observe patient movement during and between the scans via the 3D camera 8 and optionally steadily check the camera function. The medical imaging device 3 may predict an approximate time for the procedure and transmit this time to the control unit 1, in particular to facilitate a seamless workflow. On the other hand, the control unit 1, may provide MWL information of the recognized patient, an optimal monitoring slice position and/or information about patient movement to the medical imaging device, which might for example adapt the scanning parameters accordingly. The contrast medium injector 7 may provide temperature information, in particular of the contrast medium, to the control unit 1. A connection between the control unit 1 and the injector 7 may further be used to evaluate injection information to rate the injection quality, e.g. whether a pressure limit is exceeded, or whether there are irregularities caused for example by an extravasation or air bubbles. The control unit 1 may send a control signal to the injector 7 in order to initiate a contrast medium warming. Furthermore, the control unit 1 may provide MWL information of the recognized patient 9, about the injection site and/or about the patient 9, e.g. height, weight, body mass index, and/or a body surface area, and or for contrast volume scaling to the injector 7. Furthermore, the control unit may be configured to send a stop signal regarding the injection, e.g. in case of a detected extravasation at the patient 9.

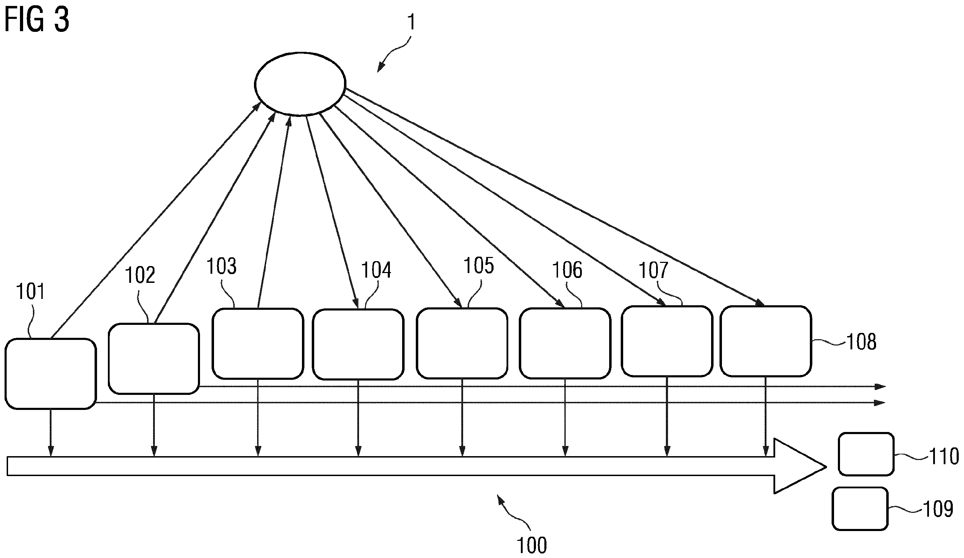

Figure 3 shows a diagram of a method according to an embodiment of the invention. At the bottom of the diagram, a time axis 100 is depicted which marks the approximate time course of the workflow. The steps of the method are either controlled or monitored by the control unit 1 or the comprise sending information to the control unit 1. Step 101 comprises monitoring the system environment and the system components, in particular by a camera and/or by other detecting devices.

This monitoring information is transferred to the control unit. Step 102 comprises monitoring the patient 9, in particular acquiring images of the patient with at least one camera, and transferring the images to the control unit 1 for interpretation in order to detect at least one aspect of the patient's condition. Both the monitoring of the system 101 and the monitoring of the patient 102 may be continued during the whole workflow, wherein different aspects may be monitored at different times. The patient may be detected via the camera in step 103, e.g. by comparing image data of the patient 9 with a patient data base 4. Based on the acquired images the control unit 1 may retrieve patient information in step 104, in particular health related information. In step 105 a scout scan may be acquired by the scanner of the medical imaging device 3. In the case of a computed tomography system, this step may in particular comprise automatically acquiring a topogram with a low radiation dose, wherein the radiation dose may in particular be determined based on image data of the patient acquired with the camera. Furthermore, this step may comprise pre-monitoring by acquiring a single-slice image of a patient's body part and determining a region of interest. In step 106 scan parameters are determined by the control unit 1 based on the patient's condition and/or the status of the system's components. Furthermore, an amount and an administration time of the contrast medium is determined in step 107. Both, the scan parameters and the parameters regarding the contrast medium are then transferred in step 108 to the injector 7 and the medical imaging device 3, respectively. Finally, in step 109, the contrast medium may be administered and, in step 110, the scan may be performed.

## Claims

1. A method for performing a medical workflow comprising a diagnostic scan of a body part of a patient (9) with a medical imaging system (2), in particular a computed tomography system, comprising a scanner, a control unit (1) and at least one camera, in particular a 3D camera, the method comprising the steps:
(a) automatically monitoring the status of at least some of the system's components and transferring said status information to the control unit (1);
(b) acquiring images of the patient (9) with the at least one camera and interpreting the images by the control unit (1) in order to detect at least one aspect of the patient's condition;
(c) determining scan parameters for the diagnostic scan based on the at least one aspect of the patient's condition and/or the status of the system's components;
(d) automatically determining a suitable amount of a contrast medium required for the diagnostic scan and/or an administration time of the contrast medium with respect to the scan, based on the at least one aspect of the patient's condition, by the control unit (1);
(e) transferring the determined scan parameters from the control unit (1) to the scanner for performing the diagnostic scan.

2. The method according to claim 1, wherein the method includes a step of the control unit (1) controlling a contrast injector (7) to inject contrast medium into the patient at the determined amount and/or administration time.

3. The method according to any one of the preceding claims, wherein the at least one camera acquires images of the patient's environment throughout the scanning session, and wherein the monitoring comprises checking for deviations from a predetermined scanning workflow and, in the case of detected deviations, sending an alarm to a user and/or initiating counter measures and/or stopping the workflow.

4. The method according to any one of the preceding claims, wherein the monitoring comprises checking for an extravasation and, in the case of detecting an extravasation stopping the workflow and/or outputting an alarm.

5. The method according to any one of the preceding claims, wherein the patient (9) is identified by visually detecting the patient (9) via the at least one camera and by comparing the visuals of the patient (9) to a data base (3) .

6. The method according to any one of the preceding claims, comprising retrieving patient information comprising health parameters from a patient data base (3) by the control unit (1), wherein the patient information is automatically provided to a user, wherein optionally suggestions for precautious actions are provided to the user.

7. The method according to any one of the preceding claims, comprising retrieving patient information from a patient data base (3) by the control unit (1), wherein the patient information comprises at least one of the patient's height, weight, body surface area, body volume, body mass index and size of the body part, in particular an organ size, and wherein said patient information is used in determining the amount of the contrast medium.

8. The method according to any one of the preceding claims, comprising automatically performing a scout scan prior to the diagnostic scan, wherein the field-of-view and the scan parameters of the scout scan are determined based on the at least one aspect of the patient's condition and/or the status of the system's components.

9. The method according to any one of the preceding claims, the method comprising the additional steps, in particular prior to the administering of the contrast medium:
- acquiring a topogram of the body part via the scanner using a low radiation dose, and automatically determining a pre-monitoring slice position based on the topogram;
- pre-monitoring the body part by acquiring a single-slice image of the body part and determining a region of interest based on the single-slice image.

10. The method according to any one of the preceding claims, wherein a minimal radiation dose for the acquisition of a topogram is determined by evaluating images of the patient (9) obtained by the at least one camera.

11. The method according to any one of the preceding claims, wherein the scan rate of the diagnostic scan is adjusted according to different phases of contrast medium invasion into the part of the patient's body.

12. A medical imaging system, in particular a computed tomography system, adapted to carry out a medical workflow comprising a diagnostic scan of a body part of a patient (9), in particular according to the method of one of the preceding claims, the system comprising:
- a control unit (1) in communication with at least some of the other components of the system, wherein the control unit (1) is configured to monitor and control the medical workflow, and configured to provide information concerning the medical workflow and the patient (9) to a user and/or to the patient (9), wherein the control unit (1) is configured to receive and forward and/or apply user input, in particular input commands and/or input information;
- a scanner adapted to acquire images of a patient's body or of parts of a patient's body;
- at least one camera, in particular a 3D camera,
wherein the at least one camera is configured to provide images of the system environment and/or of the patient (9) to the control unit (1);
wherein the control unit (1) is configured to provide the scanner with scan relevant information and/or to control the scanner.

13. The medical imaging system (2) according to claim 12,
wherein the system further comprises a contrast injector (7) adapted to inject a contrast medium into a patient (9) at an injection site, wherein the contrast injector (7) is configured to measure the temperature of the contrast medium and send the measured value to the control unit (1),
wherein the control unit (1) is configured to control functions of the contrast injector (7) and/or provide information concerning the contrast medium injection to the contrast injector (7).

14. A control unit (1) for a medical imaging system (2), in particular a medical imaging system (2) according to one of the claims 12-13,
wherein the control unit (1) is configured to monitor and control a medical workflow, wherein the control unit (1) is configured to provide information concerning the medical workflow and a patient (9) to a user and optionally to the patient (9),
wherein the control unit (1) is configured to provide a scanner of the medical imaging system (2) with scan-relevant information, in particular scan parameters,
wherein the control unit (1) is optionally configured to control functions of a contrast injector (7) that is part of the medical imaging system (2) and/or provide information concerning a contrast medium injection to the contrast injector (7).

15. The control unit according to claim 14, wherein the control unit (1) is configured to receive images from a camera throughout the workflow, and interpret the images to monitor and control the workflow.
